# EUROPEAN PATENT APPLICATION

(11) **EP 3 932 390 A1**
(43) Date of publication of application: **05.01.2022**
(21) Application number: 20183722.6
(22) Date of filing: 02.07.2020
(51) Int. Cl.: A61K 9/00, A61K 31/00, A61K 47/02, A61K 47/10, A61K 47/34, A61K 47/38

(54) **TOPICAL GEL FORMULATION CONTAINING ASIMADOLINE**

(71) Applicant: Dr. August Wolff GmbH & Co. KG Arzneimittel, 33611 Bielefeld (DE)
(72) Inventor: Soeberdt, Michael, 33611 Bielefeld (DE); Knie, Ulrich, 32105 Bad Salzulfen (DE); Abels, Christiph, 33619 Bielefeld (DE)
(74) Representative: Ter Meer Steinmeister & Partner

(57) **Abstract**

The present invention relates to a topical gel formulation comprising N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof, in an amount of 0.01 to 5.0% by weight of the topical gel formulation, calculated as free base; a gelling agent; and a solvent system. In addition, the present invention relates to such a topical gel formulation for use as a medicament and to a pharmaceutical or cosmetic composition comprising the gel formulation. Further, the present invention relates to the non-therapeutic use of the topical gel formulation as a cosmetic, preferably for topical application on the skin of a mammal, in particular for reducing itching and/or irritation of the skin.

## Description

The present invention relates to a topical gel formulation comprising N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof and to methods for preventing and/or treating diseases of the skin and/or mucosa associated with inflammation and/or pruritus using this formulation. Further, the present invention also relates to the non-therapeutic use of the topical gel formulation as a cosmetic, in particular for reducing itching and/or irritation of the skin.

### Background of the Invention

N-Methyl-N-[(1S)-1-phenyl-2-((3S)-3-hydroxypyrrolidine-1-yl)-ethyl]-2,2-diphenylacetamide (asimadoline; EMD 61753) is a known compound which acts as a peripherally selective κ-opioid receptor (KOR) agonist. For example, EP 0 569 802 claims N-methyl-N-[(1S)-1-phenyl-2-((3S)-3-hydroxypyrrolidine-1-yl)-ethyl]-2,2-diphenylacetamide among other aryl acetamides as compounds with high affinity towards κ-opioid receptors (KOR) for us as analgesics and neuroprotectives. Because of its poor ability to cross the blood brain barrier, N-methyl-N-[(1S)-1-phenyl-2-((3S)-3-hydroxypyrrolidine-1-yl)-ethyl]-2,2-diphenylacetamide lacks the psychotomimetic effects of centrally acting KOR agonists.

EP 1 572 640 A discloses asimadoline to be effective in the treatment of gastrointestinal diseases, inflammatory and non-inflammatory diseases of the gastrointestinal tract, of the urinary system and eating disorders as well as of pain and neuropathy. Even more, EP 0 752 246 claims the use of N-methyl-N-[(1S)-1-phenyl-2-((3S)-3-hydroxypyrrolidine-1-yl)-ethyl]-2,2-diphenylacetamide for the preparation of a medicament for the treatment of inflammatory diseases of the intestine, and EP 2 561 870 claims the use of KOR agonists, especially N-methyl-N-[(1S)-1-phenyl-2-((3S)-3-hydroxypyrrolidine-1-yl)-ethyl]-2,2-diphenylacetamide, for the treatment of diarrhea-predominant and alternating irritable bowel syndrome (IBS).

EP 0 761 650 discloses a thermostable crystal modification of asimadoline and its use in the treatment of inflammatory diseases and of pain related diseases.

WO2018/119108 discloses solid dosage formulations containing asimadoline for oral administration to treat patients suffering from pruritus. WO99/32096 relates to formulations containing asimadoline that are administered transdermally using a carrier effective to permit sustained release.

In view thereof there is still need in the prior art for new asimadoline containing compositions having improved properties.

Therefore, the object underlying the present invention is the provision of novel asimadoline containing compositions with improved properties, in particular for the improved prevention and/or treatment of conditions of the skin and/or mucosa associated with inflammation and/or pruritus.

This object is achieved by the provision of the topical gel formulation of the present invention.

### Summary of the Invention

The present invention relates to a topical gel formulation (also named "topical gel" or "gel" hereinafter) comprising N-methyl-N-[(1S)-1-phenyl-2-((3S)-3-hydroxypyrrolidine-1-yl)-ethyl]-2,2-diphenylacetamide (also named "asimadoline" hereinafter) or a derivative, solvate, hydrate, or prodrug thereof or a salt thereof, a gelling agent and a solvent system. The present invention also relates to methods for preventing and/or treating diseases of the skin and/or mucosa associated with inflammation and/or pruritus using this formulation and to pharmaceutical compositions (medicaments) *per se* comprising the gel formulation. Further, the present invention also relates to the non-therapeutic use of the topical gel formulation as a cosmetic, in particular for reducing itching and irritation of the skin, and to cosmetic compositions (cosmetics) *per se* comprising the gel formulation.

In particular, it has surprisingly been found out that the topical gel formulation according to the present invention is highly effective in the prevention and/or treatment of medical and cosmetic conditions of the skin and/or mucosa associated with inflammation (medical) and/or pruritus (medical) and/or itching and irritation of the skin (cosmetic). Moreover, the inventive gel formulation provides both for an increased stability (i.e. a gel formulation that maintains its stable gel structure and clear gel appearance over time without crystallization of ingredients and without degradation of asimadoline) and for an improved cosmetic acceptance (i.e. exhibiting moisturizing and skin soothing effects with good spreadability, fast absorption and without leaving a sticky feeling on the skin) leading to a well-balanced conveniently applicable product with high cosmetic acceptability and favorable skin care characteristics. Also surprisingly, the gel formulation exhibits excellent local tolerability and systemic safety often not observed with common asimadoline compositions (e.g. local and systemic side effects, such as dysphoria, sedation, polyuria or insomnia and skin irritation or even inflammation at application site, application site pain, pruritus, and stinging). In the prior art it was not possible to sufficiently fulfill these properties all together at the same time.

### Detailed Description of the Invention

According to the invention N-methyl-N-[(1S)-1-phenyl-2-((3S)-3-hydroxypyrrolidine-1-yl)-ethyl]-2,2-diphenylacetamide or a derivative or prodrug thereof can be present in the form of their free bases or as pharmaceutically and/or cosmetically (physiologically) acceptable salts, or in the form of their solvates, in particular their hydrates.

The "pharmaceutically and/or cosmetically acceptable salts" can be base addition salts or acid addition salts.

Base addition salts include salts of the compounds according to the invention with inorganic bases, such as alkali metal hydroxides, alkaline earth metal hydroxides, or with organic bases, such as mono-, di- or triethanolamine, primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, and tromethamine.

Acid addition salts include salts of the compounds according to the invention with inorganic acids, such as hydrochloric acid, sulfuric acid or phosphoric acid, or with suitable organic carboxylic or sulfonic acids or with amino acids. These are chosen, for example, from the group comprising chlorides, bromides, iodides, hydrochlorides, hydrobromides, sulfonates, methanesulfonates, sulfates, hydrogen sulfates, sulfites, hydrogen sulfites, phosphates, nitrates, methanoates, acetates, propionates, lactates, citrates, glutarates, maleates, malonates, malates, succinates, tartrates, oxalates, fumarates, benzoates, p-toluenesulfonates and/or salts of amino acids, preferably the proteinogenic amino acids. The hydrochloride is particularly preferred.

According to the invention N-methyl-N-[(1S)-1-phenyl-2-((3S)-3-hydroxypyrrolidine-1-yl)-ethyl]-2,2-diphenylacetamide can be present in the form of its derivatives and prodrugs.

"Derivative" in the present invention means any compound that is derivable from N-methyl-N-[(1S)-1-phenyl-2-((3S)-3-hydroxypyrrolidine-1-yl)-ethyl]-2,2-diphenylacetamide by a small number of chemical reactions, preferably by one or two reaction steps only (e.g. by acid addition, esterification or etherification at the 3-hydroxypyrrolidine group). Particularly preferred are the derivatives comprising an acid covalently bonded at the 3-hydroxypyrrolidine group. The acid preferably is selected from monobasic carboxylic acids, dibasic carboxylic acids and hydroxymonobasic carboxylic acids (such as disclosed in EP 1 572 640 B).

The term "prodrug" means any precursor compound that is metabolized into N-methyl-N-[(1S)-1-phenyl-2-((3S)-3-hydroxypyrrolidine-1-yl)-ethyl]-2,2-diphenylacetamide. Preferred prodrugs comprise an ester group or an ether group at the 3-hydroxypyrrolidine group.

It is known that the compound N-methyl-N-[(1S)-1-phenyl-2-((3S)-3-hydroxypyrrolidine-1-yl)-ethyl]-2,2-diphenylacetamide exhibits different crystal form types (polymorphs) including crystal form type II (see DE 42 15 213 A1) and type IV (see DE 195 31 464 A1). Type II provides a water solubility of 1.16 g/100 ml, while type IV is the thermodynamically more stable form having a lower water solubility of 0.76 g/100 ml. All crystal forms of N-methyl-N-[(1S)-1-phenyl-2-((3S)-3-hydroxypyrrolidine-1-yl)-ethyl]-2,2-diphenylacetamide can be used according to the present invention, however, crystal form IV is the preferred one because of its higher thermodynamic stability. It is further preferred not to use a mixture of different polymorphs in order to avoid reduction or even failure in efficacy resulting from different solubilities of asimadoline crystals of different crystal forms. Thus, it is most preferred to use asimadoline purely or exclusively in crystal form IV in the topical gel formulation of the present invention.

The topical gel formulation according to the invention comprises N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof in an amount of 0.01 to 5.0% by weight of the topical gel formulation, calculated as free base (i.e. calculated on the weight of N-methyl-N-[(1S)-1-phenyl-2-((3S)-3-hydroxypyrrolidine-1-yl)-ethyl]-2,2-diphenylacetamide). Preferably, the topical gel formulation according to the invention comprises N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof in an amount of 0.05 to 1.5% by weight of the topical gel formulation, calculated as free base, more preferably in an amount of 0.075 to about 1.25% by weight of the topical gel formulation, calculated as free base, and most preferably in an amount of 0.1 to about 1.0% by weight of the topical gel formulation, calculated as free base. Particularly suitable values out of these ranges are amounts of 0.1, 0.3, 0.5, 0.8, 1.0 and 1.25% by weight of the topical gel formulation, calculated as free base. Most preferred is an amount of 1.0% by weight of the topical gel formulation, calculated as free base. If the amount is below the minimum values of the indicated ranges, the treatment and/or prevention is ineffective. On the other hand, if the amount is above the maximum values mentioned, CNS side effects are observed and crystals of active ingredients (e.g. asimadoline) are formed. In other words, within the ranges as mentioned it is possible to provide stable and uniform gel formulations having a viscosity that allows an effective and convenient topical application on the skin.

The topical gel formulation according to the invention may comprise, next to N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof, one or more additional active compounds. These compounds may serve to increase efficacy, decrease one or more side effects, or decrease the required dose of N-methyl-N-[(1S)-1-phenyl-2-((3S)-3-hydroxypyrrolidine-1-yl)-ethyl]-2,2-diphenylacetamide to be administered.

Suitable additional active compounds may include steroids, antihistamines, topical anesthetics, topical retinoids, acitretin, anti-inflammatory agents, hydrocortisone, betamethasone, triamcinolone, vitamin D-analogues, estrogens, benzoyl peroxide, JAK-inhibitors, antiseptics, antihistamines, hydroxyzine, diphenhydramine, immunomodulators, cyclosporine, methotrexate, tacrolimus, pimecrolimus, azathioprine, mycophenolate, antiviral agents, acyclovir, antibiotics, cephalexin, penicillin and derivatives thereof, clindamycin, mupirocin, octenidine, trimethoprim, sulfamethoxazole, non-steroidal anti-inflammatory drugs, TNF-alpha blockers such as certolizumab pegol, etanercept, adalimumab, infliximab, golimumab, interleukin 12/23 antagonists, ustekinumab, interleukin 17-A antagonists, secukinumab, crisaborole, and apremilast. Other active compounds also known to be suitable for treating conditions of the skin and/or mucosa (i.e. skin, vaginal and oral mucosa) associated with inflammation and/or pruritus or irritation and itching, respectively, such as atopic dermatitis, psoriasis, and other skin conditions associated with pruritus or itching, may also be used in combination with asimadoline according to the present invention.

The additional active compounds may be administered in a separate pharmaceutical/cosmetic composition or may be included with N-methyl-N-[(1S)-1-phenyl-2-((3S)-3-hydroxypyrrolidine-1-yl)-ethyl]-2,2-diphenylacetamide in a single topical gel formulation as described herein. The one or more additional active compounds may be administered simultaneously with, prior to, or after administration of the topical gel formulation according to the present invention.

The topical gel formulation of the invention comprises a gelling agent (also named gel forming agent). In general, all gelling agents known in the pharmaceutical and/or cosmetic field to provide for stable gel formulations can be used according to the invention. It is preferred to use hydrophilic gelling agents. Examples of suitable gelling agents are natural gelling agents, such as pectin, agarose, gelatine and casein, or modified natural gelling agents, such as cellulose derivatives including methyl cellulose, hydroxymethyl cellulose, hydroxyethyl cellulose (HEC), hydroxymethylpropyl cellulose (HPMC) and carboxymethyl cellulose, or full synthetic gelling agents, such as polyvinylalcohols, poly(meth)acrylic acids, polyacrylamide, polyvinylpyrrolidone, polypropylene glycol, polyethylene glycol and poloxamers. Cellulose derivatives and poloxamers are preferred because of the increased stability and appropriate viscosity of the gels obtained. Most preferred are poloxamers for gel stability reasons and due to its function as solubilizer. Suitable poloxamers are Poloxamer 407 (e.g. trade name Pluronic F 127; Kolliphor P 407), or Poloxamer 188 (e.g. trade name Pluronic F 68; Kolliphor P 188).

The gelling agent used according to the present invention preferably provides for a gel having a convenient texture and sufficient flowability to be conveniently administered on or spread over the skin.

The gel formulation of the invention is sufficiently stable. This means that the gel formulation maintains its gel structure and in particular its clear appearance over time (preferably over at least 3 months, more preferably over at least 6 months, even more preferably 12 months, most preferably over at least 36 months at 25°C) without crystallization and degradation of asimadoline or any other ingredients.

The gelling agent is contained in the topical gel formulation of the invention in an amount of 1 to 25% by weight of the topical gel formulation. When the gelling agent is selected from the group of poloxamers, the gelling agent is preferably contained in the topical gel formulation in an amount of 17 to 24%, more preferably from 18 to 20% by weight of the topical gel formulation. With 16% poloxamer the formulation is rather a liquid than a gel, and with 25% poloxamer (in particular Poloxamer 407) the formulation becomes a solid mass with some air inclusions rendering the formulation less preferred. When the gelling agent is selected from the group of cellulose derivatives (such as methyl cellulose, hydroxymethyl cellulose, hydroxyethyl cellulose, hydroxymethylpropyl cellulose, or carboxymethyl cellulose), the gelling agent is preferably contained in the topical gel formulation in an amount of 1 to 10%, more preferably 2 to 5% by weight of the topical gel formulation. If the amount of gelling agent is higher than the mentioned maximum values, the viscosity becomes too high. As mentioned above, this leads to a sticky gel consistency which renders the uniform and convenient application on the skin impossible. In addition, air inclusions were observed within the viscous gels leading to a decreased uniformity and stability of the gel formulation. On the other hand, if the amount of gelling agent is lower than the mentioned minimum values, the viscosity becomes too low. As mentioned above, the gel structure is destroyed, and the gel becomes a liquid which also renders the uniform and convenient application on the skin impossible.

The topical gel formulation of the invention comprises a solvent system. In general, the solvent system forms the aqueous phase of the gel and should be able to properly solve the active ingredients and excipients present in the topical gel formulation and particularly to avoid crystallization of asimadoline. In addition, the solvent system must ensure a stable gel structure while at the same time avoiding a high or too low viscosity of the gel. It is preferred to use a solvent system comprising (preferably consisting of) water or a water/alcohol mixture. Preferred alcohols are ethanol and isopropanol, while ethanol being most preferred. The preferred solvent system consists of ethanol and water. Most suitably the mixture consists of ethanol and water in a weight ratio of from 1:8 to 1:5, most preferred, for gel viscosity and stability reasons, with an ethanol/water weight ratio of (about) 1:6. Also, if the total amount of ethanol is too high (e.g. higher than 10% by weight of the topical gel formulation), the gel becomes instable leading to a liquid/very low viscosity consistency being not suitable according to the present invention. The topical gel formulations of the invention may comprise glycerol and/or benzyl alcohol, however, these are not considered as parts of the solvent system according to the invention but rather as further excipients. In fact, glycerol, if present, is used as a humectant having moisturizing properties, and benzyl alcohol is used as a preservative.

The topical gel formulation according to the invention preferably has a pH value of 3.0 to 6.0, more preferably of 3.5 to 4.5. Within these ranges the stability of the gel is improved. The pH value of the gel formulation can be stabilized within these ranges by using buffer systems e.g. consisting of polyacids and their salts. Examples for such polyacids are citric acid, tartaric acid, phosphoric acid and malic acid. A preferred buffer system is citric acid/citrate (e.g. sodium citrate). Most suitably the citric acid/citrate buffer is contained in an amount of from 0.30 to 0.75% by weight of the total gel formulation, more preferred from 0.45 to 0.55% by weight, most preferred 0.5% by weight.

The topical formulations of the present invention may comprise further excipients such as those being suitable for pharmaceutical and/or cosmetic compositions. In particular, the further excipients that may be used according to the invention are known to the skilled person. These include, for example, one or more of a preservative, a pH adjusting agent, a humectant, a permeation enhancer; a skin care compound; an inorganic salt, diluents, wetting agents, dyestuffs, lubricants, salts for influencing the osmotic pressure, binders, detergents, solubilizers, fillers, bioadhesives, bactericides, surfactants, colorants, thickeners, softening agents, moisturizing agents, lipids, waxes, polymers or other suitable components of a gel formulation.

Examples for preservatives and/or bactericides are organic acids like formic acid, sorbic acid and benzoic acid. In addition, esters of p-hydroxybenzoic acid, formaldehyde-releasing agents like DMDM hydantoin, imidazolidinylurea or methyl chloroisothiazolinone, methylisothiazolinone, dibromodicyanobutane, iodopropynyl butylcarbamte, phenoxyethanol or benzyl alcohol can be used as preservatives/bactericides.

The compounds according to the invention are commercially available e.g. from Sigma Aldrich or BOC Sciences. Alternatively, they can be prepared as disclosed in DE 196 47 538 A1, EP 0752 246 A, and EP 0761 650 A.

The topical gel formulation of the present invention can be used as a pharmaceutical composition/medicament, as a medical device or as a cosmetic composition, depending on the indication to be treated and the respective regulatory requirements. The pharmaceutical and cosmetic compositions comprise the gel formulation and include one or more pharmaceutically and/or cosmetically acceptable excipients as mentioned above.

In the present invention, the gel formulation is preferably a dermatological composition suitable to be applied topically on the skin/epithelium of a mammal. The form of the composition is a clear gel.

Clinical disorders/indications that may be treated with the gel formulation may be selected from the group consisting of diseases of the skin and/or epithelia associated with inflammation and/or pruritus, preferably selected from diseases of stratified epithelia and underlying layers (skin, vaginal and oral epithelium) associated with inflammation and/or pruritus, e.g. edema, hyperproliferative skin diseases, dry skin conditions, inflammation due to insect bites, inflammation due to wound healing, psoriasis, psoriatic arthritis, eczema, scleroderma and other fibrotic diseases, contact dermatitis, alopecia areata, lichen planus, allergic diseases characterized by mast cell involvements, acne, rosacea, systemic lupus erythematous, urticaria, lymphoma, atopic dermatitis, seborrheic dermatitis, perioral dermatitis, vulvovaginitis, vulvodynia, oral mucositis, glossitis, gingivitis, inflammation of the hair follicle.

Non-therapeutic (cosmetic) use of the topical gel formulation of the present invention includes the topical application of the gel formulation on the skin of a mammal in individuals who experience inconvenient itching, irritation, pruritus and/or red appearance of the skin.

The gel formulation of the present invention is applied topically on the skin/epithelia of a mammal.

The gel formulation of the present invention preferably is topically applied onto the skin/epithelia of a mammal 1-5 times a day, more preferably 1-2 times a day, or when needed once the diseases is improved.

As used herein, an effective amount of N-methyl-N-[(1S)-1-phenyl-2-((3S)-3-hydroxypyrrolidine-1-yl)-ethyl]-2,2-diphenylacetamide (asimadoline) or a derivative, solvate, hydrate, or prodrug thereof or a salt thereof essentially means an amount that is effective to exhibit biological activity, preferably wherein the biological activity is defined as an effective prevention/treatment of diseases of the skin and/or mucosa associated with inflammation and/or pruritus, at the site(s) of activity in a mammalian subject. This is intended to exclude undue adverse side effects (in particular centrally mediated side effects, such as dysphoria, sedation, polyuria or insomnia etc.).

The effective amount of N-methyl-N-[(1S)-1-phenyl-2-((3S)-3-hydroxypyrrolidine-1-yl)-ethyl]-2,2-diphenylacetamide (asimadoline) or a derivative, solvate, hydrate, or prodrug thereof or salt thereof, based on the weight relative to N-methyl-N-[(1S)-1-phenyl-2-((3S)-3-hydroxypyrrolidine-1-yl)-ethyl]-2,2-diphenylacetamide, is typically from about 1.0 mg to 200 mg, more preferably from 2.0 mg to 160 mg, even more preferably 4.0 to 80 mg, most preferably from 5.0 to 40 mg (for example 5, 7, 10, 12, 15, 18, 20, 25, 28, 30, 32, 35, 38 or 40 mg) topically administered per day. Preferably the topical formulation of the present invention is administered one time per day. However, according to the invention administration is possible up to several times per day (e.g. 1, 2, 3 or 4 times per day). Above the maximum values the risk of undesired side-effects significantly increases.

The topical gel formulations according to the invention are prepared in a suitable way known to the skilled person. Preferably the topical gel formulations are prepared by first dissolving the active compound (i.e. asimadoline) in the solvent system, mixing the solution with the further excipients, if present, and adding the gelling agent to the solution under stirring at ambient temperature to obtain the uniform and stable gel formulation.

The following non-limiting examples describe the present invention in more detail.

### Example 1

The topical formulation of this example contained the following compounds in the indicated amounts. The formulation was prepared by first dissolving asimadoline in the solvent system (water), mixed with the further excipients (propylene glycol, phenoxyethanol), using a conventional magnetic stirrer (RCT basic, IKA-Labortechnik, Janke & Kunkel), then adjusting to a pH value of 3.7 by using ortho phosphoric acid 0.1 % and adding the gelling agent (Hypromellose; HPMC) to the solution under homogenizing at 80°C to obtain a uniform and stable gel formulation. The gel is opalescent and has a pH value within the range of 3.5-4.5.

| | |
|---|---|
| Asimadoline HCl | 0.300 g |
| Water, purified | 90.960 g |
| | |
| | |
| Propylene glycol | 3.000 g |
| Phenoxyethanol | 1.000 g |
| H₃PO₄ 0.1 % | 2.740 g |
| | |
| Hypromellose | 2.000 g |
| | |
| | **100.000 g** |

### Example 2

The topical formulation of this example contained the following compounds in the indicated amounts. The formulation was prepared by first dissolving asimadoline in a mixture of the solvent system (water) and the further excipients (citric acid/citrate, propylene glycol, benzyl alcohol) using a conventional magnetic stirrer (RCT basic, IKA-Labortechnik, Janke & Kunkel) under application of ultrasound (Ultra Sonic Bandelin Sonorex), if needed for complete dissolution, and adding the gelling agent (Natrosol 250 M Pharma) to the solution under homogenizing at ambient temperature to obtain a uniform and stable gel formulation. The gel is opalescent and has a pH value within the range of 3.5-4.5.

| | |
|---|---|
| Asimadoline HCl | 0.544 g |
| Water, purified | 85.856 g |
| Benzyl alcohol | 1.000 g |
| Propylene glycol | 10.000 g |
| Citric acid, anhydrous | 0.320 g |
| Sodium citrate | 0.280 g |
| | |
| Natrosol 250 M Pharma | 2.000 g |
| | |
| | **100.000 g** |

### Example 3

The topical formulation of this example contained the following compounds in the indicated amounts. The formulation was prepared by first dissolving asimadoline in a mixture of the solvent system (water) and the further excipients (citric acid/citrate, glycerol, PEG-6 caprylic/capric glycerides, benzyl alcohol) using a conventional magnetic stirrer (RCT basic, IKA-Labortechnik, Janke & Kunkel) under application of ultrasound (Ultra Sonic Bandelin Sonorex), if needed for complete dissolution, and adding the gelling agent (Poloxamer 407) to the solution under stirring at ambient temperature to obtain a uniform and stable gel formulation. The gel is clear and has a pH value within the range of 3.5-4.5.

| | |
|---|---|
| Asimadoline HCl | 0.762 g |
| Water, purified | 71.738 g |
| Benzyl alcohol | 1.000 g |
| Glycerol | 5.000 g |
| PEG-6 caprylic/capric glycerides | 3.000 g |
| Citric acid, anhydrous | 0.320 g |
| Sodium citrate | 0.180 g |
| | |
| Poloxamer 407 | 18.000 g |
| | |
| | **100.000 g** |

### Example 4

The topical formulation of this example contained the following compounds in the indicated amounts. The formulation was prepared by first dissolving asimadoline in a mixture of the solvent system (water/ethanol; about 6:1 weight ratio) with the further excipients (citric acid/citrate, glycerol, benzyl alcohol) using a conventional magnetic stirrer (RCT basic, IKA-Labortechnik, Janke & Kunkel) under application of ultrasound (Ultra Sonic Bandelin Sonorex), if needed for complete dissolution, and adding the gelling agent (Poloxamer 407) to the solution under stirring at ambient temperature to obtain a uniform and stable gel formulation. The gel is clear and has a pH value within the range of 3.5-4.5.

| | |
|---|---|
| Asimadoline HCl | 1.088 g |
| Water, purified | 59.412 g |
| Benzyl alcohol | 1.000 g |
| Glycerol | 10.000 g |
| Ethanol 96% | 10.000 g |
| Citric acid, anhydrous | 0.320 g |
| Sodium citrate | 0.180 g |
| | |
| Poloxamer 407 | 18.000 g |
| | |
| | **100.000 g** |

### Stability

Stability of the gel formulation of the Examples 1-4 was tested. The gel formulation was considered sufficiently stable if it maintained its gel structure over time, in particular up to 36 months at 25°C, without crystallization and degradation of asimadoline or any other ingredients. The highest stability was determined for Examples 3 and 4 that were stable over 36 months at 25°C. Example 2 was sufficiently stable for at least 6 months at 25°C, and Example 1 was sufficiently stable for at least 3 months at 25°C and 50°C.

## Claims

1. A topical gel formulation comprising
a) N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof, in an amount of 0.01 to 5.0% by weight of the topical gel formulation, calculated as free base;
b) a gelling agent; and
c) a solvent system.

2. The topical gel formulation according to claim 1, wherein the solvent system comprises water or a water/alcohol mixture.

3. The topical gel formulation according to claim 1 and/or claim 2, further comprising at least one of a preservative; a pH adjusting agent; a humectant; a permeation enhancer; a skin care compound; and/or an inorganic salt.

4. The topical gel formulation according to any one of claims 1 to 3, wherein the gelling agent is a hydrophilic polymer selected from the group consisting of pectins, agarose, gelatin, casein, methyl cellulose, hydroxymethyl cellulose, hydroxyethyl cellulose, hydroxymethylpropyl cellulose, carboxymethyl cellulose, polyvinylalcohols, poly(meth)acrylic acids, polyacrylamide, polyvinylpyrrolidone, polypropylene glycol, polyethylene glycol and poloxamers.

5. The topical gel formulation according to claim 4, wherein the gelling agent, when selected from poloxamers, is contained in an amount of 17 to 24% by weight of the topical gel formulation, and wherein the gelling agent, when selected from methyl cellulose, hydroxymethyl cellulose, hydroxyethyl cellulose, hydroxymethylpropyl cellulose, carboxymethyl cellulose, is contained in an amount of 1 to 10% by weight of the topical gel formulation.

6. The topical gel formulation according to any one of claims 1 to 5, wherein the N-methyl-N-[(1S)-1-phenyl-2-((3S)-3-hydroxypyrrolidine-1-yl)-ethyl]-2,2-diphenylacetamide or a derivative, solvate, hydrate, or prodrug thereof or a salt thereof is contained in an amount of 0.075 to 1.25% by weight of the topical gel formulation, calculated as free base.

7. The topical gel formulation according to any one of claims 1 to 6, wherein the pH of the formulation is adjusted to a value of 3.0 to 6.0, preferably 3.5 to 4.5.

8. The topical gel formulation according to any one of claims 1 to 7 for use as a medicament.

9. The topical gel formulation for use according to claim 8, wherein the clinical disorder to be treated is selected from the group consisting of diseases of the skin and/or epithelia associated with inflammation and/or pruritus, preferably selected from diseases of stratified epithelia and underlying layers associated with inflammation and/or pruritus, comprising edema, hyperproliferative skin diseases, dry skin conditions, inflammation due to insect bites, inflammation due to wound healing, psoriasis, psoriatic arthritis, eczema, scleroderma and other fibrotic diseases, contact dermatitis, alopecia areata, lichen planus, allergic diseases **characterized by** mast cell involvements, systemic lupus erythematous, urticaria, lymphoma, atopic dermatitis, seborrheic dermatitis, perioral dermatitis, acne, rosacea, vulvovaginitis, vulvodynia, oral mucositis, glossitis, gingivitis, inflammation of the hair follicles.

10. The topical gel formulation for use according to claim 8 and/or claim 9, wherein the gel formulation is topically applied onto the skin of a mammal, preferably 1-4 times a day.

11. Pharmaceutical composition comprising the gel formulation according to any one of claims 1 to 7 and one or more pharmaceutically acceptable excipients.

12. Cosmetic composition comprising the gel formulation according to any one of claims 1 to 7 and one or more cosmetically acceptable excipients.

13. Non-therapeutic use of the topical gel formulation according to any one of claims 1 to 7 as a cosmetic for topical application on the skin of a mammal, preferably for reducing itching and/or irritation of the skin.
